# EUROPEAN PATENT SPECIFICATION

(11) **EP 3 380 841 B1**
(45) Date of publication and mention of the grant of the patent: **02.10.2019**
(21) Application number: 16801263.1
(22) Date of filing: 25.11.2016
(51) Int. Cl.: G01N 33/542, C12Q 1/68

(54) **AUTONOMOUS SENSING MOLECULES (ASM)**
AUTONOME ABTASTMOLEKÜLE (ASM)
MOLÉCULES DE DÉTECTION AUTONOME

(30) Priority: 27.11.2015 EP 15196819
(43) Date of publication of application: 03.10.2018
(73) Proprietor: AIT Austrian Institute of Technology GmbH, 1210 Wien (AT)
(72) Inventor: BARISIC, Ivan, 1150 Vienna (AT)
(74) Representative: Gassner, Birgitta
(86) International application number: PCT/EP2016/078860
(87) International publication number: WO 2017/089571

(56) References cited:
- US-A1- 2014 051 090
- AUDREY SASSOLAS ET AL: "Homogeneous assays using aptamers", THE ANALYST, vol. 136, no. 2, 1 January 2011 (2011-01-01), pages 257-274, XP055296594, GB ISSN: 0003-2654, DOI: 10.1039/C0AN00281J
- HYOYOUNG MUN ET AL: "Homogeneous assay of target molecules based on chemiluminescence resonance energy transfer (CRET) using DNAzyme-linked aptamers", BIOSENSORS AND BIOELECTRONICS, vol. 58, 1 August 2014 (2014-08-01), pages 308-313, XP055296564, NL ISSN: 0956-5663, DOI: 10.1016/j.bios.2014.02.008 cited in the application
- EUN-JUNG JO ET AL: "Detection of ochratoxin A (OTA) in coffee using chemiluminescence resonance energy transfer (CRET) aptasensor", FOOD CHEMISTRY, vol. 194, 18 August 2015 (2015-08-18), pages 1102-1107, XP055296584, NL ISSN: 0308-8146, DOI: 10.1016/j.foodchem.2015.07.152
- GUANGFENG WANG ET AL: "Photoinduced electron transfer (PET) based label-free aptasensor for platelet-derived growth factor-BB and its logic gate application", BIOSENSORS AND BIOELECTRONICS, vol. 63, 1 January 2015 (2015-01-01), pages 552-557, XP055296585, NL ISSN: 0956-5663, DOI: 10.1016/j.bios.2014.07.067
- ZHOU ZHAOJUAN ET AL: "A general approach for rational design of fluorescent DNA aptazyme sensors based on target-induced unfolding of DNA hairpins", ANALYTICA CHIMICA ACTA, vol. 889, 7 August 2015 (2015-08-07), pages 179-186, XP029273371, ISSN: 0003-2670, DOI: 10.1016/J.ACA.2015.06.036
- WON-BO SHIM ET AL: "Chemiluminescence competitive aptamer assay for the detection of aflatoxin B1 in corn samples", FOOD CONTROL., vol. 36, no. 1, 1 February 2014 (2014-02-01), pages 30-35, XP055296609, GB ISSN: 0956-7135, DOI: 10.1016/j.foodcont.2013.07.042

## Description

### FIELD OF THE INVENTION

The present invention relates to compositions and methods for the detection of target molecules. Specifically, the present invention relates to sensing molecules comprising an aptamer and a DNAzyme to generate a detectable signal indicative of the presence of target molecules and methods utilizing such sensing molecules.

### BACKGROUND

Diagnostic tests are essential to provide rapid and simple detection and identification of chemical and biological agents such as nucleic acids, proteins, metal ions, carcinogens, infectious agents, allergens, illicit drugs, toxins and poisons. Accurate and efficient diagnosis of infectious diseases and cancers are of particular clinical interest in order to limit spreading of the respective pathogens and to significantly improve treatment outcome due to early diagnosis.

Current diagnostic methods are often time-consuming and expensive, involving special materials, skills, instrumentation and/or processing steps such as generation of specific detection agents, for example antibodies, tedious extraction methods of a sample, long cultivation times, and/or sophisticated laboratory equipment such as chromatographs or mass spectrometers.

Since aptamer technology has been established in the early 1990s, it has been applied in various diagnostic and therapeutic applications. Aptamers are typically single-stranded oligonucleotides engineered through repeated rounds of *in vitro* selection or screened from DNA or RNA libraries through the *in vitro* systematic evolution of ligands by using exponential enrichement (SELEX). Aptamers offer several advantages such as simplicity and rapidness of engineering and production, low batch-to-batch variability, thermal stability and small size and can be selected against a broad range of targets

Aptamer-based technologies have also been used in combination with DNAzymes for signal generation and amplification in the presence of a target. DNAzymes are catalytic nucleic acid enzymes, i.e., non-protein enzymes, capable of modifying specific substrates. For example, Zhu et al. (Protein Cell 1(9):842-846 (2010)) describe a method for detection of cancer cells using two single stranded DNA chains (ssDNA). One ssDNA (ssDNA1) consists of an aptamer region targeting CCRF-CEM acute leukemia cells and a linker region, while the second ssDNA (ssDNA2) consists of a horseradish-peroxidase (HRP)-mimicking DNAzyme and a linker region which is complementary to the linker region of ssDNA1. In the presence of target cells, ssDNA1 bound to the cell surface and was then removed from the system under centrifugation, thus was no longer present in the supernatant. ssDNA2 was then added to the supernatant and formed peroxidase-active G-quadruplex with hemin, catalyzing a colorimetric reaction. In the absence of target cells, ssDNA-1 hybridized with ssDNA-2, forming double-stranded DNA, thereby blocking the formation of peroxidase-acitve G-quadruplex and inhibiting a catalytic reaction.

Mun et al. (Biosensors and Bioelectronics 58: 308-313 (2014)) designed a single stranded DNAzyme-aptamer sensor based on chemiluminescence resonance energy transfer (CRET). The structure of the engineered single-stranded DNA includes the (HRP)-mimicking enzyme, a linker and a target-specific aptamer sequence modified with a quencher dye at the 3'end. In the presence of a target, the aptamer sequence was folded due to the formation of the aptamer/analyte complex, thereby inducing the quencher dye close to the DNAzyme structure. As a consequence of the close proximity between the quencher dye and the DNAzyme, which was activated by incorporation of hemin, CRET occurred. Jo et al. (Food Chemistry; 194: 1102-1107 (2015)) describe a similar aptasensor based on chemiluminescence for the detection of ochratoxin A. Aptasensors based on an aptamer and a ribozyme were also described in US2014/051090A1.

Sun et al. (Analytica Chimica Acta 885:166-173 (2015)) report an electrochemical aptamer cytosensor for detection of human liver cancer cells. A high-affinity thiolated TLS11a aptamer, covalently attached to a gold electrode, and a G-quadruplex/hemin/aptamer-AuNPs-HRP nanoprobe, consisting of a G-quadruplex/hemin/aptamer and horseradish-peroxidase (HRP) modified gold particles, were used to generate an aptamer-cell-nanoprobes sandwich-like superstructure on a gold electrode surface. Both HRP and G-quadruplex/hemin HRP mimicking DNAzyme could be used for enzyme catalysis and electrochemical signal amplification.

Wang et al. (Biosensors and Bioelectronics; 63:552-557(2015)) disclose aptasensors based on photoinduced electron transfer (PET) between DNA-Ag fluorescent nanoclusters und G-quadruplex/hemin complexes. The aptasensors comprise an aptamer sequence, fluorophore DNA-templated Ag nanoclusters and the G-quadruplex sequence. When no target is present, the aptasensor self-hybridizes into a hairpin-like structure with the G-rich sequence in a protective inactive configuration. In the presence of a target, conjugation with its aptamer induces a conformational change of the hairpin-like structure, releasing the G-quadruplex sequence part. Upon addition of hemin and K+, the horseradish peroxidase mimicking DNAzyme is formed causing electron transfer between the DNA-Ag nanoclusters to the hemin Fe(III) center of the HRP-DNAzyme and a decrease in the fluorescence intensity of the DNA-Ag nanoclusters.

Another approach for a design of aptazyme sensors through the activation of DNazymes by aptamer target-induced unfolding of DNA hairpins is disclosed in Zhou et al. (Analytica Chimica Acta, 889:179-186(2015)). Fluorescent aptasensors comprise a DNAzyme attached at its 3'end to an aptamer and at its 5'end to a short cDNA sequence partially complementary to the aptamer to enable formation of a DNA hairpin. The DNAzyme is constrained by the hairpin and cannot bind to its substrate, thus its activity is inhibited. In the presence of a target, the aptamer binds same, causing the unfolding of the hairpin, activation of the DNAzyme and cleaving of its substrate, resulting in a fluorescent signal.

An assay based on competitive binding of an aptamer-HRP-DNAzyme between an immobilized target and a target in solution is disclosed in Shim et al. (Food Control: 36(1):30-35 (2014)). If no target is present in a solution, the aptamer-HRP-DNAzyme binds to the immobilized target, causing a fluorescent signal when substrate for the DNAzyme is added to the immobilized target. If a target is present in the solution, the aptamer-HRP-DNAzyme binds to same, bound target is then removed from the solution in a washing step. Since no aptamer-DNAzyme is bound to the immobilized target, adding a substrate for the DNAzyme does not cause a fluorescent signal.

An overview of various detection assays based on aptamers is provided by Sassolas et al. (The Analyst, 136(2):257-274 (2011)).

However, all available tests, including those currently under development, are dependent on instrumentation, enzymatic reactions and sophisticated prosessing.

Thus, there is still a great need for the development of a simple, stable, cost effective and specific diagnostic tool for rapid detection of a target molecule.

An automonous and simple sensing molecule that could accurately and efficiently detect or screen for chemical and biological agents would therefore provide a significant cost and time benefit.

### SUMMARY

The invention is defined in the claims. Provided herein is a sensing molecule comprising an aptamer, a linker, and a DNAzyme, which DNAzyme is covalently bound to the aptamer via the linker and wherein in the absence of a target the DNAzyme is associated with the aptamer, preferably via hydrogen bonds, and inactive and in the presence of a target the DNAzyme is active, and wherein the aptamer is partially reverse complementary to the DNAzyme.

In some embodiments, in the absence of a target molecule the aptamer of the sensing molecule is associated with the DNAzyme and the DNAzyme is inactive. Specifically, the aptamer and the DNAzyme of the sensing molecule are directly associated, preferably via hydrogen bonds and/or optionally via further interactions such as Van der Waals forces, polar-nonpolar interactions, Pi-interactions, and/or indirectly associated via helper oligonucleotides. In some embodiments, the linker enables association of the aptamer and the DNAzyme in the absence of a target.

The DNAzyme of the sensing molecule folds into an enzymatically active structure upon binding of the aptamer to the target. A DNAzyme of the sensing molecule can be designed to mimic a reporter enzyme, preferably an alkaline phosphatase (AP), a horseradish peroxidase (HRP), a luciferase, or a glucose oxidase (GOx). Specifically, the DNAzyme is a HRP-mimicking enzyme, preferably a HRP-mimicking G-quadruplex DNAzyme.

In some embodiments, the aptamer of the sensing molecule is a DNA or a RNA aptamer. Specifically, the aptamer comprises at least 25 nucleotides, preferable at least 50 nucleotides, more preferable 25 to 75 nucleotides. In some embodiments, the aptamer comprises at least one chemically modified nucleotide or nucleotide analog. Optionally, the aptamer comprises at least one nucleotide or nucleic acid analogue with one or more covalently attached amino acid residue(s) and/or one or more amino acid analog(s). In some embodiments the aptamer comprises at least one chemically modified nucleotide or nucleotide analog, preferably any one of at least 40%, 50%, or 60% (e.g. at least 50%) of the nucleotides and/or nucleotide analogs are chemically modified In some embodiments, one or more amino acid analogs (e.g. 1, 2, 3, 4 or 5) are bound to the chemically modified nucleotide/nucleotide analog.

In some embodiments, the linker of the sensing molecule comprises at least 3 nucleotides, preferably 3 to 100 nucleotides. In some embodiments, the linker comprises a self-complementary sequence and/or comprises a sequence partially reverse complementary to the aptamer. In some embodiments, the DNAzyme, preferably part of the DNAzyme, is reverse-complementary to the aptamer (e.g. with a sequence identity of at least any one of 50%, 60%, 70%, 80, 90% or 95%). Part of aptamer sequence is reverse-complementary (e.g. with a sequence identity of at least any one of 50%, 60%, 70%, 80, 90% or 95%) and part of the aptamer sequence is reverse complementary to (a part of) the DNAzyme (e.g. with a sequence identity of at least any one of 50%, 60%, 70%, 80, 90% or 95%).

In some embodiments, the sensing molecule provided herein is a single-stranded DNA molecule.

In some embodiments, the target of the sensing molecule is selected from the group consisting of metal ions, small organic molecules, organic dyes, peptides, proteins, surface proteins, lipopolysaccharides, whole cells and microorganisms.

Further provided herein is a method for detecting a target molecule in a sample, comprising
(i) contacting the sensing molecule described herein with the sample;
(ii) determining the enzymatic activity of the DNAzyme, and
(iii) detecting the target molecule, wherein enzymatic activity is indicative of the presence of the target.

Specifically, the sample is an industrial or environmental sample, preferably a sample from soil, water, or waste-streams; or a biological sample, preferably from body fluids, swabs or tissues. As herein described, the enzymatic activity is determined by a colorimetric, fluorescent or electrochemical signal in the method for detecting a target molecule provided herein. In certain instances of the disclosure, the enzymatic activity produces a detectable colorimetric signal.

Further provided herein is use of a sensing molecule described herein for diagnosing a disorder.

### BRIEF DESCRIPTION OF THE DRAWINGS

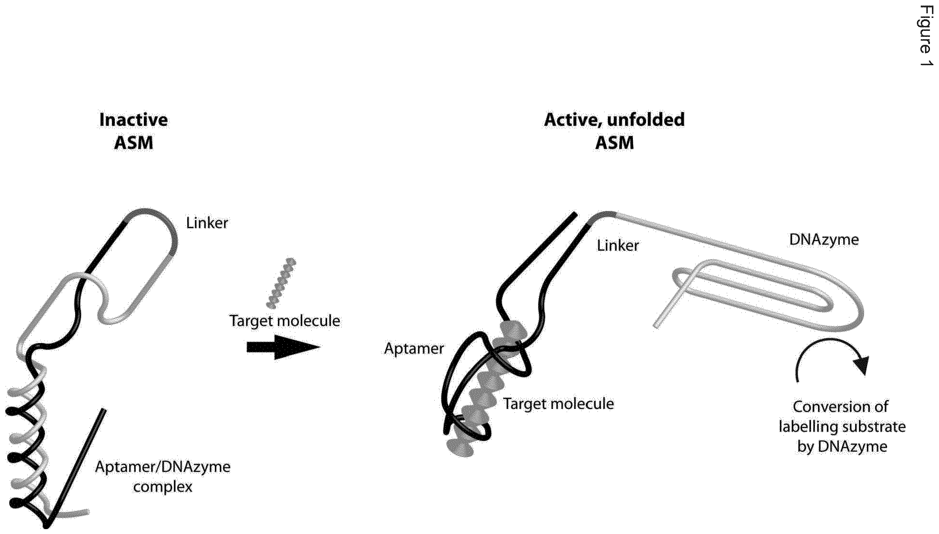

Figure 1. Schematic concept of the Autonomous Sensor Molecule (ASM) detection principle. In the inactive form of the ASM, the aptamer (black) binds to the DNAzyme (light grey) via a linker (dark grey) and blocks any activity. The ASMs are designed that the affinity of the aptamer towards a target molecule is significantly higher than to the DNAzyme. Thus, upon incubation with a target, the aptamer binds to the target, the ASM refolds and releases the catalytically active domain converting chemical substrates. This results in a detectable signal.

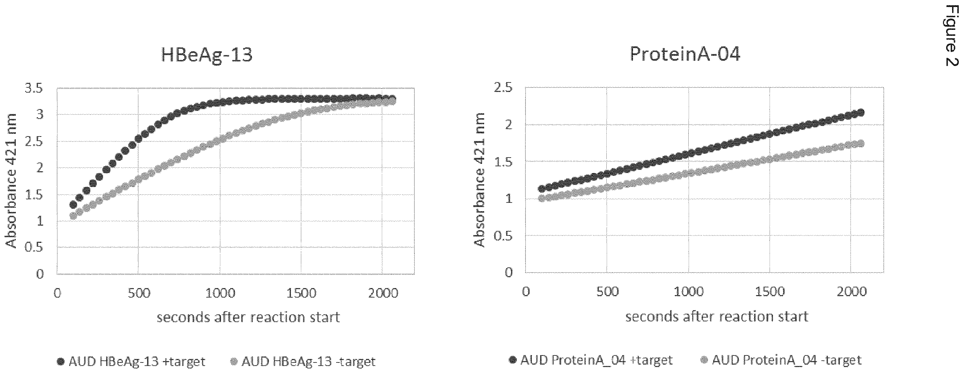

Figure 2: Graph showing increased oxidation for sensing molecules targeting (left) HbeAg (SEQ ID NO:5) or (right) Protein A (SEQ ID NO:4) in the presence of the respective target. Absorbance of reactions at 421 nm measured in seconds after H₂O₂ addition. The reaction including the target (HBeAg or Protein A, respectively) is indicated by "+target", the reaction without the target is indicated by "-target".

### DETAILED DESCRIPTION OF THE INVENTION

Specific terms as used throughout the specification have the following meaning.

A "molecule" as used herein refers to a single entity or particle made up of atoms by covalent bonds. The molecule can comprise different parts, which are made up of one single class of substances or a combination thereof. Classes of substances are, for example, single-stranded DNA (ssDNA), double-stranded DNA (dsDNA), single-stranded RNA (ssRNA), double-stranded RNA (dsRNA), including DNA and/or RNA with chemical modifications, a peptide or other organic compound. The molecule may be of varying length, size or molecular weight, and can have any activity known and/or desired by the skilled person. A "sensing molecule" refers to a molecule comprising an aptamer, a linker and a DNAzyme.

The term "nucleic acid molecule", as used herein, is intended to include DNA molecules, RNA molecules, oligonucleotides, polynucleotides and analogs or derivatives thereof. A nucleic acid molecule may be single stranded (ss) or double stranded (ds). Where single stranded, the nucleic acid molecule can be the sense strand or the antisense strand. The term includes synthetic nucleic acid molecules, such as chemically synthesized or recombinantly produced nucleic acid molecules.

As used herein, the terms "nucleic acid", "polynucleotide", and "polynucleic acid" are used interchangeably and refer to a polymeric form of nucleotides of any length, either deoxyribonucleotides or ribonucleotides, chemically modified forms or analogs of nucleotides, and combinations of the foregoing. The term includes both sense and/or anti-sense strands of RNA, cDNA, genomic DNA, synthetic forms and mixed polymers thereof. The term also includes any topological conformation, including single-stranded (ss), double-stranded (ds), partially duplexed, triplexed, hairpinned, circular, and padlocked conformations.

The term "chemically modified nucleotide" refers to nucleotides and/or nucleotide analogs, which differ in their chemical structure from conventional nucleotides and/or nucleotide analogs, having modifications in the chemical structure of the base, sugar and/or phosphate. Nucleotides can be modified at any position on their structure.

Chemically modified bases refer to nucleotide bases such as, for example, adenine, guanine, cytosine, thymine, and uracil, xanthine, hypoxanthine, isocytosine, isoguanine, inosine, and queuosine that have been modified by the replacement or addition of one or more atoms or groups such as 5-position pyrimidine modifications, 8-position purine modifications, modifications at cytosine exocyclic amines, and substitution of 5-bromo-uracil. Exemplary types of nucleotide modifications with respect to the base moieties, include, but are not limited to, alkylated, halogenated, thiolated, aminated, amidated, or acetylated bases, in various combinations as well as bases with one or more covalently bound amino acid residues and/or amino acid analogs. In case more than one amino acid residues and/or amino acid analog residues are bound to the nucleotide, only the first amino acid or amino acid analog residue is bound to the nucleotide and the further amino acid or amino acid analog residu(s) are bound to said first or any of the further amino acid or amino acid analog residue(s) to form a linear or branched chain of residues.

Chemically modified nucleotides also include nucleotides and/or nucleotide analogs which are modified with respect to the sugar moiety, as well as nucleotides having sugars or analogs thereof that are not ribosyl. For example, the sugar moieties may be, or be based on, mannoses, arabinoses, glucopyranoses, galactopyranoses, 4-thioribose, and other sugars, heterocycles, or carbocycles. Modifications of the sugar moiety, e.g. 2'-position sugar modifications, include, but are not limited to, sugar-modified ribonucleotides in which the 2'-OH is replaced by a group such as an H, OR, R, halo, SH, SR, NH₂, NHR, NR₂, or CN, wherein R is an alkyl moiety (i.e., saturated linear or branched hydrocarbon group including, for example, methyl, ethyl, isopropyl, t-butyl, heptyl, dodecyl, octadecyl, amyl, 2-ethylhexyl, and the like.). Nucleic acids containing one or more carbocyclic sugars are also included within the definition of nucleic acids.

Chemically modified nucleotides or nucleotide analogs are also meant to include nucleotides with non-natural phosphodiester internucleotide linkages such as methylphosphonates, phosphorothioates, phosphorodithioates, phosphoramides, phosphoramidates, phosphotriesters, in particular alkylesters, phosphoramidites, O-methylphosphoramidite linkages as well as peptide nucleic acid backbones. Nucleotide modifications can occur also in changes in the stereochemistry (a-nucleotide phophodiester) or by attaching different 5 '-terminal groups for example such as psoralen and derivatives, phenandroline and derivatives, ellipicitine and derivatives, EDTA, 5'-p(*N*-2-chloroethyl-*N*-methylamino)benzyl-amide, acridine and derivatives.

The term "amino acid" refers to natural amino acids, unnatural amino acids and amino acid analogs, all in their D and L stereoisomers if their structure allows such stereoisomeric forms. Natural amino acids include alanine (Ala), arginine (Arg), asparagine (Asn), aspartic acid (Asp), cysteine (Cys), glutamine (Gin), glutamic acid (Glu), glycine (Gly), histidine (His), isoleucine (Ile), leucine (Len), lysine (Lys), methionine (Met), phenylalanine (Phe), proline (Pro), serine (Ser), threonine (Thr), tryptophan (Trp), tyrosine (Tyr) and valine (Val). Unnatural amino acids include, but are not limited to azetidinecarboxylic acid, 2-aminoadipic acid, 3-aminoadipic acid, beta-alanine, aminopropionic acid, Z-aminobutyric acid, 4-aminobutyric acid, 6-aminocaproic acid, 2-aminoheptanoic acid, 2-aminoisobutyric acid, 3-aminoisobutyric acid, 2-aminopirnelic acid, 2,4-diaminoisobutyric acid, desmosine, 2,2'-diaminopimelic acid, 2,3-diaminopropionic acid, *N*-ethylglycine, *N*-ethylasparagine, hydroxylysine, allo-hydroxylysine, 3-hydroxyproline, 4-hydroxyproline, isodesmosine, allo-isoleucine, N-methylglycine, *N*-methylisoleucine, *N*-methylvaline, norvaline, norleucine, ornithine and pipecolic acid.

The term "amino acid analog" refers to natural and unnatural amino acids which are chemically blocked, reversibly or irreversibly, or modified on their N-terminal amino group or their side-chain groups to another functional group, as for example, methionine sulfoxide, methionine sulfone, S-carboxymethyl-D-cysteine, S-carboxymethyl-cysteine sulfoxide and S-carboxymethyl-D-cysteine sulfone. For example, aspartic acid-(beta-methyl ester) is an amino acid analog of aspartic acid; N-ethylglycine is an amino acid analog of glycine; or alanine carboxamide is an amino acid analog of alanine.

The term "aptamer" as used herein refers to nucleic acids (typically DNA, RNA or oligonucleotides) that are capable of binding to a particular molecular target. Aptamers emerge from *in vitro* selections or other types of aptamer selection procedures well known in the art (e.g. bead-based selection with flow cytometry or high density aptamer arrays) when the nucleic acid is added to mixtures of target molecules. An aptamer is typically between 10 and 300 nucleotides in length. RNA and DNA aptamers can be generated from *in vitro* selection experiments such as SELEX (Systematic Evolution of Ligands by Exponential Enrichment). Examples of aptamer uses and methods for making/selecting aptamers are described, for example, in Chu et al., 2006, Nucl. Acids Res. 34:e73, US 2006/0014172, US 5,840,867, US 6,001,648, US 6,225,058, US 6,207,388, and US 2002/0001810.

As used herein, the term "affinity" refers to the strength of a non-random interaction between two molecules or two parts of a molecule (e.g. parts of the same molecule) and can be expressed quantitatively as a dissociation constant (K_{D}). Binding affinity (i.e., K_{D}) can be determined using standard techniques.

The term "DNAzyme" refers to a DNA molecule that catalyzes a chemical or biological reaction. The DNAzyme may be covalently linked with one or more other molecules yet remain a DNAzyme. The DNAzyme may be single or double stranded DNA molecule. The DNAzyme may require one or more co-factor(s) for its catalytic activity.

The term "linker" as used herein refers to a material that connects two parts or moieties of a molecule. The linker may be, for example, a nucleic acid, a protein, a polypeptide, a polymer, an organic compound or a combination thereof. It may occur naturally or be produced synthetically. The linker may be bound covalently to the two parts or moieties.

The term "hairpin" refers to a stem-loop structure. The stem results from two sequences of nucleic acid or modified nucleic acid annealing together to generate a duplex. The loop is a single stranded region that lies between the two strands comprising the stem.

The term "target" refers to any target molecule for which an aptamer exists or can be generated for, and can be any organic or inorganic molecule, naturally occurring or artificially created.

As used herein, the term "associate" refers to the process in which at least two molecules or parts of a molecule (e.g. parts of the same molecule) reversibly interact with each other. The term also intends to refer to a condition of proximity between entities. The association(s) may be non-covalent, e.g., wherein the juxtaposition is energetically favored by hydrogen bonding, van der Waals interactions or electrostatic interactions, or the association(s) may be covalent. The association may be direct or indirect via additional helper oligonucleotide.

The term "helper oligonucleotide" refers to a single or double stranded oligonucleotide (DNA or RNA), which associates partially with at least two different molecules or at least two different parts of a molecule (e.g. parts of the same molecule), thereby enabling association of these molecules or parts of the molecule.

The term "complementarity" or "complementary" as used herein refers to the formation or existence of hydrogen bond(s) between one nucleic acid sequence and another nucleic acid sequence by either traditional Watson-Crick or other non-traditional types of bonding as described herein. The term "complementarity/ complementary" as used herein includes "reverse complementarity/reverse complementary". Perfect complementary means that all the contiguous residues of a nucleic acid sequence will form hydrogen bond with the same number of contiguous residues in a second nucleic acid sequence. Partial complementarity can include various mismatches or non-based paired nucleotides (e.g., 1, 2, 3, 4, 5, 6, 7, 8, 9, 10 or more mismatches, non-nucleotide linkers, or non-based paired nucleotides) within the nucleic acid molecule, which can result in bulges, loops, or overhangs between the two nucleic acid sequences. Such partial complementarity can be represented by a % complementarity that is determined by the number of non-base paired nucleotides, i.e., about 50%, 60%, 70%, 80%, 90% etc. within the total number of nucleotides involved.

The term "hybridize" or "anneal" refers to the ability of completely or partially complementary nucleic acid strands to come together under specified hybridization conditions in a parallel or preferably antiparallel orientation. The nucleic acid strands interact via hydrogen bonding between bases on opposing strands and form a stable or quasi-stable double-stranded helical structure or may result in the formation of a triplex, or other higher-ordered structure. Although hydrogen bonds typically form between adenine and thymine or uracil (A and T or U) or cytosine and guanine (C and G), other base pairs may be formed (e.g., Adams et al., The Biochemistry of the Nucleic Acids, 11th ed., 1992). The ability of two nucleotide sequences to hybridize with each other is based on the degree of complementarity of the two nucleotide sequences, which in turn is based on the fraction of matched complementary nucleotide pairs. The more nucleotides in a given sequence that are complementary to another sequence, the more stringent the conditions can be for hybridization and the more specific will be the binding of the two sequences. Increased stringency is achieved by elevating the temperature, increasing the ratio of co-solvents, lowering the salt concentration, and the like.

As will be appreciated by persons skilled in the art, stringent conditions are sequence-dependent and are different in different circumstances. For example, longer fragments may require higher hybridization temperatures for specific hybridization than short fragments. Because other factors, such as base composition and length of the complementary strands, presence of organic solvents and ions, and the extent of base mismatching, may affect the stringency of hybridization, the combination of parameters can be more important than the absolute measure of any one parameter alone. In some embodiments, hybridization can be made to occur under high stringency conditions, such as high temperatures or 0.1X SCC. Examples of high stringent conditions are known in the art; see e.g., Sambrook et al., Molecular Cloning: A Laboratory Manual, 2d Edition, 1989, and Short Protocols in Molecular Biology, ed. Ausubel et al. In general, increasing the temperature at which the hybridization is performed increases the stringency. As such, the hybridization reactions described herein can be performed at a different temperature depending on the desired stringency of hybridization. Hybridization temperatures can be as low as or even lower than 5°C, but are typically greater than 22°C, and more typically greater than about 30°C, and even more typically in excess of 37°C. In other embodiments, the stringency of the hybridization can further be altered by the addition or removal of components of the buffered solution. In some embodiments, hybridization is permitted under medium stringency conditions. In other embodiments, hybridization is permitted under low stringency conditions. In some embodiments, a nucleic acid sequence is perfectly complementary to another nucleic acid with which it binds. In other embodiments, one or more mismatches are present between the hybridized molecules or hybridized portions of molecules.

The term "hydrogen bond" as used herein, refers to a form of association between an electronegative atom and a hydrogen atom attached to a second atom exceeding the electronegativity of carbon. The electronegative-atom having a free electron pair to share with the hydrogen atom is the so-called hydrogen bond acceptor, and may be nitrogen, oxygen, sulfur or fluorine. The hydrogen atom bound to the electronegative atom is generally referred to as a hydrogen bond donor. The terms electronegative and electropositive as used herein will be readily understood by the person skilled in the art to mean the tendency of an atom to attract the pair of electrons in a covalent bond so as to lead to an unsymmetrical distribution of electrons and hence the formation of a dipole moment. The hydrogen bond is stronger than a van der Waals interaction, but weaker than covalent or ionic bonds.

The term "electrostatic interaction", as used herein, refers to any interaction occurring between charged components, molecules or ions, due to attractive forces when components of opposite electric charge are attracted to each other. Examples include, but are not limited to: ionic interactions, covalent interactions, interactions between an ion and a dipole (ion and polar molecule), interactions between two dipoles (partial charges of polar molecules), hydrogen bonds and London dispersion bonds (induced dipoles of polarizable molecules).

The term "covalent bond" or "covalent interaction" refers to bonds or interactions created by the sharing of a pair of electrons between atoms. Covalent bonds/interactions include, but are not limited to atom bonds, homopolar bonds, σ-σ-interactions, σ-π-interactions, two-electron-to-center bonds, single bonds, double bonds, triple bonds, as well as combinations of these interactions/bonds. The mentioned interactions/bonds, can be polar or polarized, or can be non-polar or non-polarized.

"Non-covalent" refers to associations between atoms and molecules such as ionic interactions (e.g., dipole- dipole interactions, ion pairing, and salt formation), hydrogen bonding, non-polar interactions, inclusion complexes, clathration, van der Waals interactions (e.g., pi-pi stacking), and combinations thereof.

"Enzymatically active" refers to the ability to measurably catalyze a biological reaction. Enzymatic activity can be measured by methods and assays known in the art including, but not limited to, methods and assays based on a detectable signal such as chemical or physical signals.

"Enzymatically inactive" refers to the inability to catalyze a biological or chemical reaction or inability to adopt a conformation in order to catalyze a biological or chemical reaction, even in the proximity of any cofactors, substrates or other elements required for the reaction.

A "reporter enzyme" as used herein refes to an enzyme capable of activating a reaction that results in a detectable signal, i.e. is a signal generating enzyme. The reaction may require a specific substrate, which is any chemical species susceptible to a detectable change upon action of the reporter enzyme.

A "detectable signal" refers to a physical or chemical signal, which can be measured by visual or instrumental methods, and includes colorimetric, fluorescent chemiluminescent, and chemoelectrical signals.

The term "sample" refers to a composition that will be subjected to analysis that is suspected of containing the target of interest. A sample for analysis may be in a liquid form (e.g., an aqueous mixture) or solid and subsequently dissolved in an aqueous mixture. A sample may be from any source, such as an industrial sample from a waste-stream, an environmental sample such as soil or water, food or a biological sample, such as body fluids (e.g. blood, urine, saliva, or sputum), swabs, liquor cerebrospinalis, stool or tissue. A sample may be a derivative of an industrial, environmental or biological sample, such as an extract, a dilution, a filtrate, or a reconstituted precipitate.

It is an object of the invention to provide a sensing molecule comprising different parts, i.e. an aptamer, a linker and a DNAzyme as defined in the claims. Specifically, the aptamer is covalently bound to the DNAzyme via the linker. In some embodiments, the linker connects the 3' end of the aptamer with the 5'-end of the DNAzyme. For example, the 3'-end of the aptamer is connected to the linker (e.g. the 5'-end of a nucleic acid linker or N-terminal end of a peptide linker) and the 5'-end of the DNAzyme is connected to the linker (e.g. the 3'-end of a nucleic acid linker or C-terminal end of a peptide linker). In some embodiments, the linker connects the 3'-end of the DNAzyme with the 5'-end of the aptamer. For example, the 3' end of the DNAzyme is connected to the linker (e.g. the 5'-end of a nucleic acid linker or N-terminal end of a peptide linker) and the 5'-end of the aptamer is connected to the linker (e.g., the 3'-end of a nucleic acid linker or C-terminal end of a peptide linker).

The sensing molecule described herein may be a single-stranded nucleic acid molecule, a double-stranded nucleic acid molecule or a combination thereof, e.g. one part of the molecule (e.g. the aptamer and/or the DNAzyme) is single-stranded and another part of the molecule (e.g. the linker) is double-stranded. The sensing molecule described herein may be a DNA molecule, an RNA molecule, or a combination thereof. For example, the sensing molecule may comprise a single-stranded DNA or RNA sequence as aptamer, a single-stranded or double-stranded DNA or RNA as linker, and a single-stranded or double-stranded DNA as DNAzyme. Specifically, the sensing molecule may be a combination of ssDNA, dsDNA, ssRNA, dsRNA, and a peptide or organic compound. For example, the sensing molecule may comprise a single-stranded DNA or RNA sequence as aptamer, a peptide or organic compound as linker and a single or double stranded DNA sequence as DNAzyme.

In some embodiments, the sensing molecule is a nucleic acid molecule with at least one chemically modified nucleotide or nucleotide analog (e.g. a nucleotide/ nucleotide analog comprising at least any one of 1, 2, 3, 4, 5, 6, 7, 8, 9 or 10 amino acid residue(s) and/or amino acid analog(s), wherein the first amino acid or amino acid analog residue is bound to the nucleotide/nucleotide analog (e.g. covalently bound), preferably via a cross-linker and, optionally, any further residues are directly or indirectly bound to said first residue). In some embodiments, any one of at least 5, 10, 15, 20, 25, 30, 40, or 50% of the nucleotides/nucleotide analogs within the sensing molecule are chemically modified. In some embodiments, the sensing molecule is a nucleic acid molecule with at least one chemically modified nucleotide or nucleotide analog within the aptamer sequence (e.g. any one of 40%, 50% or 60% of the nucleotides within the aptamer sequence are chemically modified), wherein one amino acid or amino acid analog residue is bound to the nucleotide/nucleotide analog. (e.g. via a cross-linker). In some embodiments, the sensing molecule is a nucleic acid molecule with at least one chemically modified nucleotide or nucleotide analog within the aptamer sequence (e.g. any one of 40%, 50% or 60% of the nucleotides within the aptamer sequence are chemically modified), wherein two amino acid or amino acid analog residues are bound to the nucleotide/nucleotide analog (one residue bound to the nucleotide via a cross-linker and the second residue bound to the first residue).

### Aptamer

Aptamers can bind to various molecular targets and are viewed as complements to antibodies. Aptamers have found applications in many areas, such as biotechnology, medicine, pharmacology, microbiology, and analytical chemistry, including chromatographic separation and biosensors. Coupled with their ability to bind a variety of targets, the robust nature of oligonucleotides, in terms of synthesis, storage, and wide range of temperature stability and chemical manipulation, makes them highly suitable for biosensor design and engineering.

Aptamers are nucleic acids which can be used for targeting various organic and inorganic materials. Once an aptamer which specifically binds to a certain material is isolated, it can be consistently reproduced at low costs using automated oligomer synthesis methods. In some embodiments, the aptamer is a single DNA, RNA or derivative or analog thereof, specifically a single-stranded DNA. In some embodiments, the aptamer is a single-stranded nucleic acid molecule with secondary structures that facilitate high-affinity binding to a target.

In some embodiments, the aptamer comprises any one of at least 10, 15, 25, 50, 75, 100, or 150 nucleotides and/or nucleotide analogs, specifically at least 25 nucleotides/nucleotide analogs. In some embodiments, the aptamer comprises at least 50 nucleotides and/or nucleotide analogs. In some embodiments, the aptamer comprises from 25 to 75 nucelotides and/or nucleotide analogs.

In some embodiments, the aptamer comprises at least one chemically modified nucleotide/nucleotide analog. In some embodiments, any one of at least 5, 10, 15, 20, 25, 30, 40, 50, 60, 70, 80, 90 or 95% of the nucleotides and/or nucleotide analogs within an aptamer are chemically modified. In some embodiments, at least any one of 40%, 50% or 60% of the nucleotides and/or nucleotide analogs are chemically modified (e.g. have at least one amino acid or amino acid analog residue bound to it). In some embodiments, at least 50% of the nucleotides and or nucleotide analogs of the aptamer sequence are chemically modified (e.g. have at least one amino acid or amino acid analog residue bound to it). Chemical modifications may be in the structure of the base, sugar and/or phosphate. Specifically, chemically modified nucleotides and/or nucleotide analogs are modifications of the nucleotide base (e.g. such as 5-position pyrimidine modifications, 8-position purine modifications). In some embodiments, the nucleotide bases are modified by (e.g. covalently) binding one or more amino acid residue and/or one or more amino acid analog residue to the base (e.g. at least any one of 1, 2, 3, 4, 5, 6, 7, 8, 9, or 10 amino acid residue(s) and/or amino acid analog(s)). If more than one amino acid or amino acid analog residues are attached to a nucleotide/nucleotide analog, only the first residue is directly and (e.g., covalently) bound to the nucleotide and any further residues are bound either directly to the first amino acid or amino acid analog residue or indirectly (via the second, third etc. residue), forming a linear or branched chain of amino acid or amino acid analog residues). In some embodiments, at least one nucleotide/nucleotide analog (e.g. at least 40%, 50%, or 60% of the aptamer sequence) is chemically modified, wherein at least one (e.g. one or two) amino acid or amino acid analog residue(s) are bound to the nucleotide (e.g. via a cross-linker). In some embodiments, the aptamer comprises a phosphoramidite backbone (e.g. at least any one of 10%, 20%, 30%, 40%, 50%, 60%, 70%, 80%, 90%, or 100% of the aptamer sequence have a phosphoramidite backbone).

Chemical modification of the nucleotide/nucleotide analogs with amino acid/amino acid analogs may be as follows: For example, any one of 1, 2, 3, 4, 5, 6, 7, 8, 9, 10, 11, 12, 13, 14, or 15 amino acid or amino acid analog residues may be bound to the nucleotide/nucleotide analog. In some instances, any one of 1 to 5, 5 to 15, 10 to 30 or even up to 50 amino acid or amino acid analog residues may be bound. If more than one residue is bound, the amino acid residues are bound as dipeptide or a linear or branched oligopeptide or polypeptide via the first residue. In some embodiments, the first amino acid or amino acid analog residue of such chemically modified nucleotide is bound via a cross-linker.

In order to introduce chemical modification "click chemistry" may be used. In general terms, "click chemistry" describes reactions used to join small chemical subunits in a modular fashion, yielding singular reaction products that are typically physiologically stable and stereospecific. Click chemistry applications make use of azide alkyne Huisgen cycloaddition, a two-step process that uses quantitative chemical reactions of alkyne and azide moieties to create covalent carbon-heteroatom bonds between biochemical species.

Other typical cross-linkers or attachment chemistries used for attaching a molecule (e.g. an amino acid/amino acid analog) to a nucleic acid molecules include but are not limited to biotins, (e.g. Biotin dT, Biotin-TEG), amino modifiers (e.g. 5' Amino Modifier C6, 5' Amino Modifier C12, Amino Modifier dT, Uni-Link™ Amino Modifier), azide (NHS esters), alkynes (e.g. 5'Hexenyl, 5'Octadinynyl dU), and thiol modifiers (e.g. dithiol, dithiol phsphoramidite (DPTA)). In some embodiments, the cross linker is an azide/NHS ester. In some embodiments, the cross linker is an alkyne, e.g. 5-Octadinynyl dU.

Biotins are frequently used in attachment chemistry. 5' Biotin is a versatile linker. 5' Dual Biotin inserts two adjacent biotin moieties in a sequence, which can slightly increase affinity to streptavidin. Biotin dT allows placement of a biotin internally without disrupting nucleotide spacing. Biotin-TEG helps reduce steric hindrance in applications that require the use of magnetic beads.

Amino Modifiers provide an alternative attachment chemistry. Primary amines are reactive with a number of useful molecules such as isothiocyanates, NHS esters, or activated carboxylates. The amino-modifier 5' Amino Modifier C6 with a spacer arm of 6-7 atoms is the simplest choice. 5' Amino Modifier C12 increases the distance between the functional amine and the DNA sequence. Amino Modifier dT inserts the functionality internally from an added dT base while the Uni-Link™ Amino Modifier does so without an additional nucleotide.

Azide (NHS Ester) modifications use an NHS ester functional group to attach an azide moiety at the 5', 3', or any internal position in an oligonucleotide. This azide moiety may subsequently be used to attach alkyne modified groups using the click reaction.

Alkyne modifiers are used to react with azide-labeled functional groups to form stable bonds through the click reaction. 5' Hexynyl is the simplest and most popular way to introduce a 5' terminal alkyne group. 5-Octadinynyl dU is a modified base with an 8-carbon linker terminating in an alkyne group and is the preferred way to insert alkynes at internal positions within a sequence, but can also be used for 3' or 5' attachment.

A thiol group can be used to attach an oligonucleotide to a variety of fluorescent and nonfluorescent moieties or surfaces. Dithiol can be inserted into an oligonucleotide at the 5' position, the 3' position or internally. Each insertion results in two SH groups available for coupling with ligands or surfaces. The dithiol phosphoramidite (DTPA) modification can be inserted in series so that 2, or even 3, groups can be positioned adjacent to each other to increase efficiency of ligand/surface interactions.

Aptamers specifically recognize and bind to a target, i.e. particularly adhere to a target in a sample but do not substantially recognize or adhere to other structurally-unrelated molecules in the sample. An aptamer of the sensing molecule described herein may have an affinity for the target, for example, with a K_{D} of at least any one of 1 µM, 100 nM, 10 nM, 1 nM, 100 pM, 10 pM, 1 pM, or less.

Modification of the relatively large DNA by using chemically modified nucleotides/nucleotide analogs within the aptamer sequence allows the formation of significantly more 3D conformations and interactions with binding partners via e.g. hydrogen bonds, Van der Waals forces, polar-nonpolar interactions, Pi-interactions, etc.

In some embodiments, the aptamer associates with the DNAzyme and/or linker of the sensing molecule described herein, specifically in the absence of a target. In some embodiments, the aptamer associates directly with or specifically and directly binds to (e.g. via hydrogen bonds) the DNAzyme and/or linker in the absence of a target. In some embodiments, the sequence of the aptamer has a complementarity (e.g. reverse complementarity) to a sequence within the DNAzyme and/or linker of at least any one of 5, 10, 20, 30, 40, 50, 60, 70 or 80% and associates or hybridizes with the DNAzyme in the absence of a target, preferably via hydrogen bonds. It is understood that such sequence complementarity is determined over the length of the aptamer. In some embodiments, the sequence of the aptamer is (partially) complementary (e.g., reverse complementary) to part of the DNAzyme (to a particular secondary structure of the sequence or intervening sequences between such secondary structures of the DNAzyme). In some embodiments, the sequence of the aptamer is (partially) reverse complementary to the DNAzyme. In some embodiments, part of the aptamer sequence is complementary (e.g. reverse complementary) to part of the DNAzyme (to a particular secondary structure of the sequence or intervening sequences between such secondary structures of the DNAzyme), with a sequence identity sufficient to allow association/hybridization of the aptamer with DNAzyme (part of the aptamer to part of the DNAzyme), preferably with a sequence identity of at least any one of 5, 10, 20, 30, 40, 50, 60, 70 or 80% over the entire length of the aptamer (e.g. a sequene identity of at least any one of 50%, 60%, 70% 80%, 90% or 95% over the part of the aptamer that is able to associate with the part of the DNAzyme). In some embodiments, the aptamer further comprises a sequence that is (partially) complementary (e.g. reverse complementary) to the linker sequence (a part or stretch of the linker sequence) with a sequence identity sufficient to allow association of the aptamer with the linker (part of the linker), preferably with a sequence identity of at least any one of 5, 10, 20, 30, 40, 50, 60, 70 or 80% over the entire length of the aptamer (e.g. a sequene identity of at least any one of 50%, 60%, 70% 80%, 90% or 95% over the part of the aptamer that is able to associate with the part of the linker). In some embodiments, the aptamer sequence partially complementary with the DNAzyme and the aptamer sequence partially complementary with the linker do not overlap.

Specifically, the aptamer has a higher affinity to the target relative to the DNAzyme and/or linker. The differences in binding affinity to the target compared to the DNAzyme and/or linker can be at least any one of 1.5, 2, 5, 10, 50, 100, or 1000-fold.

In some embodiments, the aptamer associates indirectly with the DNAzymeand/or linker in the absence of a target. Specifically, the aptamer associates with or binds to a helper oligonucleotide which also associates with the DNAzyme and/or linker (e.g. a helper oligonucleotide partially complementary /e.g., reverse complementary) to the aptamer and partially complementary (e.g. reverse complementary) to the DNAzyme and/or linker), thereby bringing the aptamer and DNAzyme in close proximity, and enabling an interaction between the aptamer and DNAzyme. Specifically, the aptamer has a higher affinity to the target relative to the helper oligonucleotide. The differences in binding affinity to the target compared to the helper oligonucleotide can be at least any one of 1.5, 2, 5, 10, 50, 100, or 1000-fold. In some embodiments, the helper oligonucleotide comprises at least anyone of 10, 12, 15, 18, 20 or 30 nucleotides and/or nucleotide analogs. In some embodiments, the helper oligonucleotide is a single-stranded DNA or RNA nucleotide. In some embodiments, the helper oligonucleotide is at least any one of at least 10, 15, 20, 30, 40 or 50% complementary (e.g. reverse complementary) to the aptamer (e.g. the 5' or 3' part of the helper oligonucleotide is complementary/reverse complementary to the aptamer) and any one of at least 10, 15, 20, 30, 40 or 50% complementary (e.g. reverse complementary) to the DNAzyme (e.g. the 3'end of the oligonucleotide is complementary/reverse complementary to the DNAzyme if the 5'end is complementary/reverse complementary to the aptamer or the 5'end of the oligonucleotide is complementary/reverse complementary to the DNAzyme if the 3'end is complementary/reverse complementary to the aptamer). It is understood that sequence complementarity is determined over the length of the helper oligonucleotide. For example, the helper oligonucleotide comprises 20 nucleotides and/or nucleotide analogs, wherein the first 10 nucleotides and/or nucleotide analogs starting from the 5'end of the oligonucleotide are complementary (e.g. at least 20% (reverse) complementary over to the length of the whole helper oligonucleotide) to the aptamer and the first 10 nucleotides and/or nucleotide analogs starting from the 3'end of the helper oligonucleotide are complementary (e.g. at least 20% (reverse) complementary over to the length of the whole helper oligonucleotide) to the DNAzyme (e.g. a sequence within the DNAzyme).

Since the binding affinity of the aptamer to the target is higher compared to the binding affinity to the helper oligonucleotide and/or DNAzyme, the aptamer dissociates from the DNAzyme and/or the helper oligonucleotide in the presence of a target. In some embodiments, the aptamer inhibits or blocks the catalytic activity of the DNAzyme in the absence of a target (e.g. by hybridizing via intramolecular hydrogen bonds to part of the sequence of the DNAzyme). In some embodiments, the aptamer inhibits or blocks the DNAzyme from adopting or forming a conformation required for its catalytic activity in the absence of a target (e.g. by hybridizing via intramolecular hydrogen bonds to part of the sequence of the DNAzyme). Such inhibition, blocking or inactivation of the DNAzyme in the absence of the target is independent of the presence or proximity of any co-factors, substrates or other elements needed for the catalytic reaction, i.e. even if all elements are present and in appropriate proximity for the catalytic reaction to occur, the DNAzyme is incapable, i.e. inactive, to perform its catalytic activity. In some embodiments, the aptamer and the DNAzyme (part of the DNAzyme forming a particular secondary structure) and/or the linker sequence are associated via hydrogen bonds, and optionally further via Van der Waals forces, polar-nonpolar interactions, Pi-interactions, thereby blocking the DNAzyme in the absence of a target.

In some embodiments, the aptamer specifically binds to a target selected from the group consisting of Protein A, Hepatitis B e antigen (HBeAg) and Carcinoembryonic Antigen (CEA). In some embodiments, the aptamer comprises a sequence selected from the goup consisting of SEQ ID NO:1-3.

### Linker

Provided herein are sensing molecules comprising a linker connecting an aptamer to a DNAzyme. In some embodiments, the linker is a sequence (e.g. a nucleic acid sequence), specifically a single-stranded DNA sequence, double-stranded DNA sequence, single-stranded RNA sequence, double-stranded RNA sequence, and derivatives or analogs thereof. In some embodiments, the linker comprises any one of at least 3, 5, 7, 10, 15, 20, 30, 40, 50, 60, 70, 80, 90, or 100 nucleotides. In some embodiments, the linker has a topological conformation which enables the association of the two moieties it connects, such as a stem-loop, pseudoknot, hairpin, loop, cruciform or triplex conformation. For example, the linker comprises a self-complementary sequence (e.g. a sequence where its 5'part is complememtary or partially complementary to its 3'part) thereby forming hydrogen bonds between the 5'linker sequence and 3' sequence, bringing the aptamer and DNAzyme in close proximity, and enabling association of the aptamer and the DNAzyme in the absence of a target. In some embodiments, the linker sequence is partially reverse complementary to the aptamer. In some embodiments the linker sequence comprises or consists of a sequence of SEQ ID NOs:6 or 7 or variants thereof with a sequence identity of at least any one of 60%, 70%, 80%, 90% or 95% to SEQ ID NOs: 6 or SEQ ID NO:7.

In some embodiments, the linker is a peptide, polypeptide, protein or protein bindig complex such as the biotin/streptavidin or avidin/streptavidin complex. In some embodiments, the linker comprises at least any one of 3, 5, 7, 10, 15, 20, 30, 40, 50, 60, 70, 80, or 90 amino acid residues and/or amino acid analogues. In some embodiments, the linker is a polymer, a crosslinker such as polyethylene glycol (PEG) or maleimidoundecanoic acid, or other organic molecules.

### DNAzyme

Provided herein are sensing molecules comprising a DNAzyme linked to an aptamer. In some embodiments, the DNAzyme is a single-stranded DNA molecule. In some embodiments, the DNAzyme is a double stranded DNA molecule.

The DNAzyme may be designed to catalyze any biological or chemical reaction. The DNAzyme may need specific co-factors for adopting an enzymatically-active conformation and/or for catalyzing a biological or chemical reaction. Specifically, such cofactor is present when the aptamer and the DNAzyme are associated with each other in the absence of the target. Exemplary enzymatic activities of the DNAzymes described herein include but are not limited to enzymatic activities of (i) oxidoreductases, catalyzing oxidation/reduction reactions, (ii) transferases, transferring a functional group (e.g., a methyl or phosphate group, (iii) hydrolases, catalyzing hydrolysis of various bonds, (iv) lyases, cleaving bonds by means other than hydrolysis and oxidation, (v) isomerases, catalyzing isomerization changes within a single molecule, and (vi) ligases, joining two molecules with covalent bonds. Specifically, the DNAzyme can be developed to mimic a reporter enzyme capable of generating and/or amplifying a detectable signal, such as a measurable chemical or physical signal (e.g. a colorimetric signal). Reporter enzymes can be an alkaline phosphatase (AP) in combination with 5-bromo-4-chloro-3-indolylphosphate (BCIP) or nitro blue tetrazolium as substrate, the horseradish peroxidase (HRP) in combination with 3,3',5,5'-tetramethylbenzidine; 3-amino-9-ethylcarbazole; 3,3'-diaminobenzidine, or 2,2'-azino-bis(3-ethylbenzothiazoline-6-sulfonic acid) diammonium salt (ABTS) and hydrogen peroxide as substrate, the luciferase in combination with D-luciferin, or the glucose oxidase (GOx) in combination with glucose, 4-aminophenazone + phenol or glucose + 2,2'-azino-bis(3-ethylbenzothiazoline-6-sulphonic acid).

Enzymatic activity of the DNAzyme may be determined by methods known in the art, in particular methods based on a detectable signal (Li et al., Chem Commun (Camb), 41:4209-11 (2007)). In this disclosure the detectable signal is a colorimetric signal, i.e. a change in color, a chemiluminescent signal or fluorescence signal, i.e. generation of light, or a chemoelectrical signal, i.e. change of pH-value. In some embodiments, the detectable signal is a colorimetric signal. In some embodiments the DNAzyme (e.g. HRP mimicking enzyme) produces a colorimetric signal (e.g. in the presence of 2,2'-azino-bis(3-ethylbenzothiazoline-6-sulfonic acid) diammonium salt (ABTS) and hydrogen peroxide as substrate).

Optionally, the DNAzyme is a HRP enzyme or HRP-mimicking enzyme such as a G-quadruplex DNAzyme. G-Quadruplex structures typically comprise a sequence motif with at least four runs of guanines (G-tracts), in which each G-tract most often contains at least three guanines at G-rich regions. Quadruplex structures with only two stacks of guanines are also possible (Bochman et al., Nat Rev Genet 13(11):770-780 (2012)). Thus, G-quadruplex DNAzymes may comprise four G-tracts (G1, G2, G3, G4), each containing three or more guanines. The intervening sequences between these G-tracts may be extruded as (single-stranded) loops.The terms HRP enzyme or HRP-mimicking enzyme are used interchangeably herein. G-quadruplex guanine-rich single-stranded hemin-binding aptamers (referred herein also as hemin/G-quadruplex DNAzymes) have been found to strongly bind hemin to form DNAzymes with peroxidase-like activity. These hemin/G-quadruplex DNAzymes effectively catalyze the H₂O₂-mediated oxidation of a substrate (e.g. 2,2'-azino-bis(3-ethylbenzothiazoline)-6-sulfonate, 4-chloro-1-haphtol(4CN) or 3,3'-diaminobenzidien) or lead to the generation of chemiluminescence in the presence of H₂O₂/luminol. As disclosed, the DNAzyme can be developed to mimic an alkaline phosphatase (AP) (also referred to herein as "AP-mimicking enzyme"), a horseradish peroxidase (HRP) ("HRP-mimicking enzyme"), a luciferase (also referred to herein as "luciferase mimicking enzyme"), or a glucose oxidase (GOx; also referred to herein as "GOx-mimicking enzyme").

Specifically, the DNAzyme as described herein is inactive in the absence of a target (despite the presence of any cofactor(s) of the DNAzyme such as e.g. hemin) and active in the presence of a target.

In the presence of a target, the aptamer dissociates from the DNAzyme. In some embodiments, the DNAzyme undergoes conformational changes upon dissociation from the aptamer. These conformational changes may be required for the activity of the DNAzyme. Specifically, the DNAzyme folds into or adopts an enzymatically active structure upon dissociation of the aptamer. In some embodiments, the DNAzyme requires co-factors (e.g. hemin) for adopting an enzymatically-active conformation and/or for catalyzing a biological or chemical reaction.

In the absence of the target, the DNAzyme is inactive, for example, due to steric hindrance of the DNAzyme (e.g. its catalytic activity or capability to undergo conformational changes) by the aptamer and/or helper oligonucleotides. Steric hindrance may result, for example, from close proximity of the aptamer/helper oligonucleotide(s), association, interaction or binding of the DNAzyme to the aptamer/helper oligonucleotide(s). In some embodiments, the DNAzyme is inhibited or blocked due to hybridization via hydrogen bonds of the aptamer, the linker and/or helper oligonucleotides to the DNAzyme, and thus, the DNAzyme cannot form a functional structure. Inhibition, blocking or inactivation of the DNAzyme in the absence of the target is independent of the presence or proximity of any co-factors, substrates or other elements needed for the catalytic reaction, i.e. even if all elements are present and in appropriate proximity for the catalytic reaction to occur, the DNAzyme is incapable, i.e. inactive, to perform its catalytic activity. In some embodiments, the DNAzyme is a G-quadruplex guanine-rich single-stranded hemin-binding aptamers, which is inhibited/blocked or inactivated in the absence of a target even if its cofactor, i.e., hemin, is present.

In some embodiments, the sequence of the aptamer is complementary (e.g., reverse complementary) to a part of the DNAzyme with a sequence identity of any one of at least 5, 10, 20, 30, 40, 50, 60, 70 or 80% over the entire length of the aptamer (e.g. a sequene identity of at least any one of 50%, 60%, 70%, 80%, 90% or 95% over the part of the aptamer that is able to associates with the part of the DNAzyme) and the DNAzyme is inhibited or blocked from adopting an enzymatically active conformation and/or for catalyzing a biological or chemical reaction by forming intramolecular hydrogen bonds between the aptamer and part of the DNAzyme.

For example, when the DNAzyme comprises a G-quadruplex structure, the aptamer may be (reverse) complementary to an intervening sequence between two G-tracts (e.g. between G1 and G2, G2 and G3, or G3 and G4) or (reverse) complementary with a sequence identity of any one of at least 5, 10, 20, 30, 40, 50, 60, 70 or 80% over the entire length of the aptamer (e.g. a sequene identity of at least any one of 50%, 60%, 70% 80%, 90% or 95% over the part of the aptamer that is able to associates with the G tracts intervening sequenc of the DNAzyme). In some embodiments, the DNAzyme comprises or consists of a sequence of SEQ ID NO: 8 or SEQ ID NO:9 or a variant thereof with a sequence identity of at least any one of 60%, 70%, 80%, 90% or 95% to SEQ ID NO: 8 or SEQ ID NO:9.

### Target

Targets for the sensing molecules described herein include, but are not limited to, proteins or portions thereof (e.g., receptors, antigen, antibodies, enzymes, growth factors), peptides, enzyme inhibitors, hormones, carbohydrates, polysaccharides, glycoproteins, lipids, phospholipids, metabolites, metal ions, cofactors, inhibitors, drugs, dyes, vitamins, nucleic acids, membrane structures, receptors, organelles, and viruses. Specifically, targets include metal ions, small organic molecules, organic dyes, peptides, proteins, surface proteins, lipopolysaccharides, whole cells and microorganisms.

In some embodiments, the target is selected from the group consisting of Protein A, Hepatitis B e antigen (HBeAg) and Carcinoembryonic Antigen (CEA).

Target molecules can be free in solution or can be part of a larger cellular structure (e.g., such as a receptor embedded in a cell membrane). In some embodiments, a target molecule is one which does not naturally bind to nucleic acids. In some embodiments, aptamers are selected which are activated by target molecules comprising molecules having an identified biological activity (e.g., a known enzymatic activity, receptor activity, or a known structural role).

In some embodiments, the target molecule does not naturally bind to nucleic acids. The target molecule may bind in a sequence-specific or non-specific manner to an aptamer. In some embodiments, a plurality of target molecules binds to the aptamer. Selection for aptamers specifically responsive to a plurality of target molecules (i.e. not activated by single targets within the plurality) may be achieved by including at least two negative selection steps in which subsets of the target molecules are provided.

In some embodiments, aptamers are selected which bind specifically to a modified target molecule but which do not bind to non-modified target molecules. Targeted modifications include, e.g., post-translational modifications of a protein, such as phosphorylation, ribosylation, methylation (Arg, Asp, N, 5, or O-directed), prenylation (e.g., faraesyl, geranylgeranyl, and the like), acetylation, acylation, allelic variations within a protein (e.g., single amino acid changes in a protein) and cleavage sites in a protein. In another embodiment, intermediates in a chemical synthesis pathway can be targeted, as well as starting and final products. In still a further embodiment, stereochemically distinct species of molecules can be targeted.

Provided herein in one aspect is a sensing molecule (e.g. single stranded DNA sensing molecule) comprising an aptamer, a linker sequence (e.g. a selfcomplementary linker sequence) and a DNAzyme (e.g. hemin/G-quadruplex DNAzymes), wherein the DNAzyme is covalently bound to the aptamer via the linker and the DNAzyme is associated with the aptamer, preferably via hydrogen bonds, and inactive (despite the presence of any cofactor(s)) in the absence of a target and active (e.g. capable of producing a detectable signal such as a colorimetric signal) in the presence of a target. In some embodiments, the aptamer is at least 60%, 70%, 80%, 90% or 95% reverse complementary to the DNAzyme (e.g. to part of the DNAzyme sequence forming a particular secondary structure or to intervening sequences of such structure) and/or linker. In some embodiments, the linker sequence is a sequence comprising the sequence of SEQ ID NO:6 or SEQ ID NO:7). In some embodiments, the DNAzyme is a hemin/G-quadruplex DNAzyme comprising a sequence of SEQ ID NO:8 or SEQ ID NO:9) which is inactive in the absence of a target despite the presence of hemin. In some embodiments, the aptamer is at least 60%, 70%, 80%, 90% or 95% reverse complementary to an intervening sequence between two G-tracts (e.g. between G1 and G2, G2 and G3, or G3 and G4) of a hemin/G-quadruplex DNAzyme (e.g., DNAzyme comprising the sequence of SEQ ID NO:8 or SEQ ID NO:9).

Provided herein in another aspect is a sensing molecule comprising an aptamer with at least one chemically modified nucleotide (e.g. a nucleotide bound to at least one amino acid or amino acid analog), a linker sequence (e.g. a sequence of SEQ ID NO:6 or SEQ ID NO:7), and a DNAzyme (e.g. hemin/G-quadruplex DNAzyme such as a DNAzyme of SEQ ID NO:8 or SEQ ID NO:9), wherein the DNAzyme is covalently bound to the aptamer via the linker and the DNAzyme is inactive (e.g. associated via hydrogen bonds to the aptamer and/or linker) in the absence of a target despite the presence of any cofactors (e.g. hemin) and active (e.g. capable of producing a detectable signal such as a colorimetric signal) in the presence of a target. In some embodiments, at least any one of 40%, 50% or 60% (e.g. 50%) of the nucleotides within the aptamer sequence is chemically modified, preferably at least one (e.g. one or two) amino acid or amino acid analog residue is bound to any modified nucleotide/nucleotide analog (e.g. via a cross-linker). In some embodiments, the aptamer is at least 60%, 70%, 80%, 90% or 95% reverse complementary to the DNAzyme (e.g. to part of the DNAzyme sequence forming a particular secondary structure or to intervening sequences of such structure) and/or linker. In some embodiments, the aptamer is at least 60%, 70%, 80%, 90% or 95% reverse complementary to an intervening sequence between two G-tracts (e.g. between G1 and G2, G2 and G3, or G3 and G4) of a hemin/G-quadruplex DNAzyme (e.g. DNAzyme comprising the sequence of SEQ ID NO:8 or SEQ ID NO:9).

### Methods

Provided herein are methods for detecting a target molecule in a sample, comprising
(i) contacting the sensing molecule described herein with the sample, optionally first contacting the sensing molecule with any cofactor(s) of the DNAzyme and then contacting the sensing molecule in the presence of any cofactor(s) of the DNAzyme with the sample;
(ii) determining the enzymatic activity of the DNAzyme, and
(iii) detecting the target molecule, wherein enzymatic activity is indicative of the presence of the target.

In some embodiments of the methods provided herein, the sensing molecules are added to the sample. In some embodiments, any cofactor(s) of the DNAzyme are added to the sensing molecule and subsequently the reaction mixture of the sensing molecule and any cofactors is added to the sample. In some embodiments, the sensing molecules are attached to a solid support (e.g. a chip, nitrocellulose, an electrode, glass slide, membrane, gel, dendrimers, etc.) and the solid support is incubated with the sample. In some embodiments of the methods provided herein, the sensing molecules are attached to a solid support, and the solid support is first incubated with any cofactors of the DNAzyme and then subsequently incubated with the sample. As herein described the enzymatic activity of the DNAzyme produces a detectable signal, preferably a colorimetric, fluorescent or electrochemical signal. In some instances of the disclosure, the detactable signal in the methods provided herein is a colorimetric signal.

In some embodiments, the sensing molecule of the methods provided herein comprises an aptamer, a linker sequence (e.g. a selfcomplementary linker sequence) and a DNAzyme (e.g. hemin/G-quadruplex DNAzymes), wherein the DNAzyme is covalently bound to the aptamer via the linker and the DNAzyme is associated with the aptamer via hydrogen bonds and inactive (despite the presence of any cofactor(s)) in the absence of a target and active (e.g. capable of producing a detectable signal such as a colorimetric signal) in the presence of a target. In some embodiments in the methods provided herein, the aptamer is at least 60%, 70%, 80%, 90% or 95% reverse complementary to the DNAzyme (e.g. to part of the DNAzyme Sequence forming a particular secondary structure or to intervening sequences of such structure). In some embodiments of the methods provided herein, the linker sequence is a sequence comprising the sequence of SEQ ID NO:6 or SEQ ID NO:7). In some embodiments of the methods provided herein, the DNAzyme is a hemin/G-quadruplex DNAzyme comprising a sequence of SEQ ID NO:8 or SEQ ID NO:9) which is inactive in the absence of a target despite the presence of hemin. In some embodiments of the methods provided herein, the aptamer is at least 60%, 70%, 80%, 90% or 95% reverse complementary to an intervening sequence between two G-tracts (e.g. between G1 and G2, G2 and G3, or G3 and G4) of a hemin/G-quadruplex DNAzyme (e.g., DNAzyme comprising the sequence of SEQ ID NO:8 or SEQ ID NO:9).

In some embodiments, the sensing molecule of the methods provided herein is a sensing molecule comprising an aptamer with at least one chemically modified nucleotide (e.g. a nucleotide bound to at least one amino acid or amino acid analog), a linker sequence (e.g. a sequence of SEQ ID NO:6 or SEQ ID NO:7), and a DNAzyme (e.g. hemin/G-quadruplex DNAzyme such as a DNAzyme of SEQ ID NO:8 or SEQ ID NO:9), wherein the DNAzyme is covalently bound to the aptamer via the linker and the DNAzyme is inactive (e.g. associated via hydrogen bonds to the aptamer and/or linker) in the absence of a target despite the presence of any cofactors (e.g. hemin) and active (e.g. capable of producing a detectable signal such as a colorimetric signal) in the presence of a target. In some embodiments, at least any one of 40%, 50% or 60% (e.g. 50%) of the nucleotides within the aptamer sequence is chemically modified, preferably at least one (e.g. one or two) amino acid or amino acid analog residue is bound to any modified nucleotide/nucleotide analog (e.g. via a cross-linker). In some embodiments, the aptamer is at least 60%, 70%, 80%, 90% or 95% reverse complementary to the DNAzyme (e.g. to part of the DNAzyme Sequence forming a particular secondary structure or to intervening sequences of such structure). In some embodiments, the aptamer is at least 60%, 70%, 80%, 90% or 95% reverse complementary to an intervening sequence between two G-tracts (e.g. between G1 and G2, G2 and G3, or G3 and G4) of a hemin/G-quadruplex DNAzyme (e.g., DNAzyme comprising the sequence of SEQ ID NO:8 or SEQ ID NO:9).

A sample may be from any source, such as an industrial or environmental sample, preferably from soil, water, food, or waste-stream or a biological sample, such as blood, urine, saliva, sputum, swabs, liquor cerebrospinalis, stool, or tissue. A sample may be a derivative of an industrial, environmental or biological sample, such as an extract, a dilution, a filtrate, or a reconstituted precipitate. In some embodiments, the sample is in a liquid form (e.g. an aqueous mixture).

Provided are further uses of the sensing molecule described herein for diagnosing a disorder. Specifically, uses of the sensing molecule for diagnosing a disorder by determining or measuring the presence or amount of a target molecule in a sample such as a biological sample. For example, use of the sensing molecule described herein for diagnosing a disorder by detecting a target molecule which is associated with said disorder.

### EXAMPLES

### Example 1: Generation of a sensing molecule

Sequence regions (BR - binding region) that block HRP-mimicking DNAzyme activity are identified using hybridization experiments with single stranded oligonucleotides targeting the HRP-mimicking DNAzyme (sequences that are complementary to the DNAzyme). Upon the hybridization of the oligonucleotides to the DNAzyme, the acitivity of the DNAzyme is determined as known in the art and described in Travascio et al. (Chem Biol., 11:779-87 (1999)).

Random aptamers comprising the complementary BR sequence are synthesized using methods known in the art. (Darmostuk et al., Biotechnology Advances, 33, 1141-1161 (2015); Radom etal., Biotechnology Advances, 31, 1260-1274 (2013); Szeitner,et al., Journal of pharmaceutical and biomedical analysis 101, 58-65 (2014)). In some emodiments, the target molecule can be a surface antigen of bacteria such as e.g. lipopolysaccharides. The length of the non-BR sequence is kept as short as possible to minimize aptamer/antigen binding outside the BR. During the following aptamer selection process, the non-BR region will be gradually increased.

Purification and selection of the synthetized aptamers is performed according to previously described procedures (Darmostuk et al., Biotechnology Advances, 33, 1141-1161 (2015); Radom et al., Biotechnology Advances, 31, 1260-1274 (2013); Szeitner et al., Journal of pharmaceutical and biomedical analysis 101, 58-65 (2014)).. In addition to the described standard protocols, aptamers are incubated during the affinity chromatography with BR oligonucleotides of the DNAzyme and in the final evaluation with the whole DNAzyme. If the affinity of the aptamer to the antigen is higher than to the BR oligonucleotide/DNAzyme, the BR oligonucleotide/DNAzyme is displaced from the aptamer and the aptamer can be purified using standard procedures such as affinity chromatography (1-3).

Suitable aptamers, i.e. aptamers that associated with the BR oligonucleotide/DNAzyme but have a higher affinity to the target molecule, e.g. a lipopolysaccharide of bacteria, are selected. Each selected aptamer is covalently connected to the DNAzyme via a DNA linker oligonucleotide that is long enough to prevent the formation of non-functional aptamers and DNAzymes due to steric hindrance and enables controlled folding/unfolding reactions. The sensing molecule may either be produced by chemical synthesis of the entire nucleic acid sensing molecule or chemical linkage of the aptamer, linker and DNAzyme sequences.

The final and purified sensing molecules, each comprising a different aptamer selected in the previous step, are evaluated in a single tube per aptamer variant comprising only a chromogenic substrate and required buffers.e.g. as described in Kong et al. (Talanta,.80(2):459-65(2009)) or Travascio et al. (Chem Biol., 11:779-87 (1999)) The sensing molecule is discarded if a color change is observed in the absence of a target in the reaction mixture. In addition, if none of the final and purified sensing molecules shows a color change in the presence of a target, novel aptamers with longer non-BR sequences are generated as described above. Subsequently, all steps are repeated until a functional sensing molecule is generated.

### Example 2:

### Oligonucleotides

The sensor molecules comprise an aptamer sequence and a DNAzyme sequence. The aptamer sequences for the proof-of-principle experiments were retrieved from literature and are targeting Protein A (Stoltenburg et al., Sci. Rep. 6, 33812 (2016)). Hepatitis B e Antigen (Huang et al., Sci. Rep. 6, 31103 (2016)) and Carcinoembryonic Antigen (Wen et al., Biosens. Bioelectron. 83, 142-148 (2016)). Different G-quadruplex sequences were designed, attaching the 3' end of the aptamer sequences given in table 1. In some cases a self-complementary spacer/linker sequence was included to enhance intramolecular binding and therefore blocking of the G-quadruplex sequence for hemin in the absence of the target of the aptamer. Bases in between the G₃ and G₄ segments are partly reverse complementary to the aptamer sequence and are expected to avoid G-quadruplex-hemin complexes. Addition of the antigen releases the G-quadruplex sequence by binding of the aptamer sequence, enabling catalysis of the oxidation of ABTS by means of H₂O₂. The reaction is subjected to colorimetric analysis.

**Table 1. Aptamer targets and sequences used for proof-of-principle experiments**

| **Aptamer target** | **Aptamer sequence** | **Reference** |
|---|---|---|
| HBeAg | GGGCGAAGACCGGGACGGGAGGATTC TGTAGATTGGTTTT (SEQ ID NO:1) | (Huang et al., 2016) |
| CEA | CCACGATACCAGCTTATTCAATTCGTGG (SEQ ID NO:2) | (Wen et al., 2016) |
| Protein A | ATACCAGCTTATTCAATTAGCAACATGA GGGGGATAGAGGGGGTGGGTTCTCTCG GCT (SEQ ID NO:3) | (Stoltenburg et al., 2016) |

### Reagents

All oligonucleotides (sensing molecules) were obtained from Integrated DNA Technologies (Leuven, Belgium). 4-(2-Hydroxyethyl)piperazine-1-ethanesulfonic acid (Hepes), Hemin (bovine), hydrogen peroxide solution, 2,2'-azino-bis(3-ethylbenzo-thiazoline-6-sulfonic acid) diammonium salt (ABTS), KCI, KAc, NaCl, Triton X-100, DMSO, Carcinoembryonic Antigen from human fluids, and Protein A from Staphylococcus aureus were purchased from Sigma Aldrich (St. Louis, Missouri). Hepatitis B e Antigen recombinant protein was purchased from Biorbyt Ltd. (Cambridge, UK).

### DNAzyme-catalyzed peroxidation in the presence of the antigen

Lyophilized oligonucleotides (sensing molecules) are dissolved in water to a concentration of 100 µM. The oligonucleotide stock solution is further diluted to 10 µM in water. The oligonucleotide solution is denatured at 95 °C for 5 min and then gradually cooled down to 25 °C in order to avoid aggregates. Per reaction, 500 nM sensing molecule is mixed with 500 nM hemin in HEPES buffer (25 mM HEPES, 20 mM KCI, 200 mM NaCl, 0.05% Triton X-100, 1% DMSO, pH 7.4) and different amounts of the antigen, which is targeted by the aptamer, are added. The reaction is incubated for 2 h to ensure binding of the antigen by the aptamer and subsequent formation of G-quadruplex-hemin complexes due to the release of the G-quadruplex sequence from the aptamer sequence in the case of antigen binding. 15 mM ABTS and 0.6 mM H₂O₂ is added to start the reaction. Absorption at 421 nm is measured as a function of time over 1.5 h with the EnSpire multimode plate reader (Perkin Elmer). Kinetic parameters can be determined by means of the Michaelis-Menten equation (Liu et al., Chem. Cent. J. 8, 43 (2014)).

Different combinations of the reagents will be used to ensure specificity of the reaction. Blank experiments are run 1.) without the sensing molecule but with hemin and the antigen; run 2.) without the antigen, but with DNAzyme and hemin.

Alternatively, the reaction is tested in Tris buffer (10 mM Tris-HAc buffer, 5 mM KAc, 0.002 % Triton X-100, pH 7.0) and in MES buffer (25 mM MES, 200 mM NaCl, 10 mM KCI, 1 % DMSO, 0.05 % Triton X-100, pH 5.1). Different concentrations of the antigen are added to the sensing molecules and the mixture is incubated at 25 °C for 20 min. 500 nM hemin is added and the mixtures are incubated for 1 h at 25 C. 3.2 mM ABTS and 2.2 mM H₂O₂ are added to start the reaction. Absorbance at 421 nm is measured (Zhou et al., Anal. Chim. Acta 678, 124-127 (2010)).

As shown in Figure 2, increased oxidation of the substrate ABTS in the presence of the respective targets is measured for sensing molecules targeting Protein A or HBeAg (Sensing molecules comprising the sequences listed in Table 2; SEQ ID NO:4 and SEQ ID NO:5, respectively).'

The linker sequences of the HBeAg and Protein A binding sensing molecules (SEQ ID NOs:4 and 5) form a stem loop and are partly reverse complementary to the respective aptamer sequences. The intramolecular stem loop and reverse complementary sequence lead to steric hindrance of the DNAzyme in absence of the target of the aptamer.

**Table 2. Aptamer-DNAzyme sensing molecules which showed increased ATBS oxidation in the presence of the target (underlined sequence is sequence forming stem loop). The Protein A sensing molecule comprises the linker sequence: TA (SEQ ID NO:6) and the DNAzyme sequence TGG GCC TGA TGG GAT AGG GGT TGG GC (SEQ ID NO:8). The HbeAG sensing molecule comprises the linker sequence GA TTA ATC AAC ACC ACT CTC CAG ACT TCT C (SEQ ID NO:7) and the DNAzyme sequence GG GTA GGG CGG GTT GGG CC (SEQ ID NO:9).**

| **target** | **5' to 3' sequence** |
|---|---|
| Protein A | ATA CCA GCT TAT TCA ATT AGC AAC ATG AGG GGG ATA GAG GGG GTG GGT TCT CTC GGC TTA TGG GCC TGA TGG GAT AGG GGT TGG GC (SEQ ID NO:4) |
| HBeAg | GGG CGA AGA CCG GGA CGG GAG GAT TCT GTA GAT TGG TTT TGA TTA ATC AAC ACC ACT CTC CAG ACT TCT CGG GTA GGG CGG GTT GGG CC (SEQ ID NO:5) |

## Claims

1. A sensing molecule comprising an aptamer, a linker, and a DNAzyme, which DNAzyme is covalently bound to the aptamer via the linker and wherein in the absence of a target the DNAzyme is associated with the aptamer and inactive and in the presence of a target the DNAzyme is active, and wherein the aptamer is partially reverse complementary to the DNAzyme.

2. The sensing molecule of claim 1, wherein the aptamer and the DNAzyme are directly associated, preferably via hydrogen bonds and/or indirectly associated via helper oligonucleotides.

3. The sensing molecule of claim 1, wherein the DNAzyme folds into an enzymatically active structure upon binding of the aptamer to the target.

4. The sensing molecule of any one of claims 1 to 3, wherein the DNAzyme is a HRP-mimicking enzyme, preferably a hemin-binding HRP-mimicking G-quadruplex DNAzyme.

5. The sensing molecule of any one of claims 1 to 4, wherein the aptamer is a DNA or a RNA aptamer.

6. The sensing molecule of any one of claims 1 to 5, wherein the aptamer comprises at least 25 nucleotides, preferable at least 50 nucleotides, more preferable 25 to 75 nucleotides and/or nucleotide analogs.

7. The sensing molecule of any one of claims 1 to 6, wherein the aptamer comprises at least one chemically modified nucleotide or nucleotide analog, preferably wherein any one of at least 40%, 50% or 60% of the nucleotides and/or nucleotide analogs are chemically modified.

8. The sensing molecule of any one of claims 1 to 7, wherein the aptamer comprises at least one nucleotide and/or nucleotide analog with one or more covalently attached amino acid residue(s) and/or one or more amino acid analog(s), preferably wherein one or two amino acid or amino acid analog residue(s) are covalently attached to the nucleotide and/or nucleotide analog.

9. The sensing molecule of any one of claims 1 to 8, wherein the linker comprises at least 3 nucleotides, preferably 3 to 100 nucleotides and/or nucleotide analogs.

10. The sensing molecule of any one of claims 1, 2, 4 to 9, wherein the linker enables association of the aptamer and the DNAzyme in the absence of a target, preferably wherein the linker comprises a self-complementary sequence.

11. The sensing molecule of any one of claims 1 to 10, wherein the sensing molecule is a single-stranded DNA molecule.

12. The sensing molecule of any one of claims 1 to 11, wherein the target is selected from the group consisting of metal ions, small organic molecules, organic dyes, peptides, proteins, surface proteins, lipopolysaccharides, whole cells and microorganisms.

13. A method for detecting a target molecule in a sample, comprising
(i) contacting the sensing molecule of any one of claims 1 to 12 with the sample;
(ii) determining the enzymatic activity of the DNAzyme, and
(iii) detecting the target molecule, wherein enzymatic activity is indicative of the presence of the target.

14. The method of claim 13, wherein the sample is an industrial or environmental sample, preferably a sample from soil, water, or waste-streams; or a biological sample, preferably from body fluids, swabs or tissues.

15. In vitro use of a sensing molecule of any one of claims 1 to 12 for diagnosing a disorder.

## Patentansprüche

1. Sensormolekül, umfassend ein Aptamer, einen Linker und ein DNAzym, wobei das DNAzym kovalent über den Linker an das Aptamer gebunden ist und wobei in Abwesenheit eines Ziels das DNAzym mit dem Aptamer assoziiert und inaktiv ist und in Anwesenheit eines Ziels das DNAzym aktiv ist, und wobei das Aptamer teilweise umgekehrt komplementär zu dem DNAzym ist.

2. Sensormolekül nach Anspruch 1, wobei das Aptamer und das DNAzym direkt assoziiert sind, vorzugsweise über Wasserstoffbrücken und/oder indirekt über Helferoligonukleotide.

3. Sensormolekül nach Anspruch 1, wobei sich das DNAzym bei Bindung des Aptamers an das Ziel zu einer enzymatisch aktiven Struktur faltet.

4. Sensormolekül nach einem der Ansprüche 1 bis 3, wobei das DNAzym ein HRP-imitierendes Enzym ist, vorzugsweise ein Hämin-bindendes HRP-imitierendes G-Quadruplex - DNAzym.

5. Sensormolekül nach einem der Ansprüche 1 bis 4, wobei das Aptamer ein DNA- oder ein RNA-Aptamer ist.

6. Sensormolekül nach einem der Ansprüche 1 bis 5, wobei das Aptamer mindestens 25 Nukleotide, vorzugsweise mindestens 50 Nukleotide, noch bevorzugter 25 bis 75 Nukleotide und/oder Nukleotidanaloga umfasst.

7. Sensormolekül nach einem der Ansprüche 1 bis 6, wobei das Aptamer mindestens ein chemisch modifiziertes Nukleotid oder Nukleotidanalogon umfasst, wobei vorzugsweise irgendeines von mindestens 40 %, 50 % oder 60 % der Nukleotide und/oder Nukleotidanaloga chemisch modifiziert ist.

8. Sensormolekül nach einem der Ansprüche 1 bis 7, wobei das Aptamer mindestens ein Nukleotid und/oder Nukleotidanalogon mit einem oder mehreren kovalent gebundenen Aminosäureresten und/oder einem oder mehreren Aminosäureanaloga umfasst, wobei vorzugsweise ein oder zwei Aminosäure- oder Aminosäureanalogonreste kovalent an das Nukleotid und/oder das Nukleotidanalogon gebunden sind.

9. Sensormolekül nach einem der Ansprüche 1 bis 8, wobei das Aptamer mindestens 3 Nukleotide, vorzugsweise mindestens 3 bis 100 Nukleotide und/oder Nukleotidanaloga umfasst.

10. Sensormolekül nach einem der Ansprüche 1, 2, 4 bis 9, wobei der Linker die Verbindung von dem Aptamer und dem DNAzym in Abwesenheit eines Ziels ermöglicht, wobei vorzugsweise der Linker eine selbstkomplementäre Sequenz umfasst.

11. Sensormolekül nach einem der Ansprüche 1 bis 10, wobei das Sensormolekül ein einzelsträngiges DNA-Molekül ist.

12. Sensormolekül nach einem der Ansprüche 1 bis 11, wobei das Ziel ausgewählt ist aus der Gruppe, bestehend aus Metallionen, kleinen organischen Molekülen, organischen Farbstoffen, Peptiden, Proteinen, Oberflächenproteinen, Lipopolysacchariden, ganzen Zellen und Mikroorganismen.

13. Verfahren zum Nachweisen eines Zielmoleküls in einer Probe, umfassend
(i) Inkontaktbringen des Sensormoleküls nach einem der Ansprüche 1 bis 12 mit der Probe;
(ii) Bestimmen der enzymatischen Aktivität des DNAzyms, und
(iii) Nachweisen des Zielmoleküls, wobei die enzymatische Aktivität auf die Anwesenheit des Ziels hinweist.

14. Verfahren nach Anspruch 13, wobei die Probe eine Industrie- oder Umweltprobe ist, vorzugsweise eine Probe aus Boden, Wasser oder Abfallströmen; oder eine biologische Probe, vorzugsweise aus Körperfluiden, Abstrichen oder Geweben.

15. *In vitro*-Verwendung eines Sensormoleküls nach einem der Ansprüche 1 bis 12 zur Diagnose einer Erkrankung.

## Revendications

1. Molécule de détection comprenant un aptamère, un lieur et une DNAzyme, laquelle DNAzyme est liée de manière covalente à l'aptamère par l'intermédiaire du lieur et dans laquelle en l'absence d'une cible, la DNAzyme est associée à l'aptamère et inactive et en présence d'une cible, la DNAzyme est active, et dans laquelle l'aptamère est partiellement l'inverse complémentaire de la DNAzyme.

2. Molécule de détection selon la revendication 1, dans laquelle l'aptamère et la DNAzyme sont directement associés, de préférence par l'intermédiaire de liaisons hydrogène et/ou indirectement associés par l'intermédiaire d'oligonucléotides auxiliaires.

3. Molécule de détection selon la revendication 1, dans laquelle la DNAzyme se plie en une structure enzymatiquement active lors de la liaison de l'aptamère à la cible.

4. Molécule de détection selon l'une quelconque des revendications 1 à 3, dans laquelle la DNAzyme est une enzyme imitant la HRP, de préférence une DNAzyme G-quadruplexe imitant la HRP et se liant à l'hémine.

5. Molécule de détection selon l'une quelconque des revendications 1 à 4, dans laquelle l'aptamère est un ADN ou un aptamère ARN.

6. Molécule de détection selon l'une quelconque des revendications 1 à 5, dans laquelle l'aptamère comprend au moins 25 nucléotides, de préférence au moins 50 nucléotides, plus préférablement de 25 à 75 nucléotides et/ou des analogues de nucléotides.

7. Molécule de détection selon l'une quelconque des revendications 1 à 6, dans laquelle l'aptamère comprend au moins un nucléotide chimiquement modifié ou un analogue de nucléotides, de préférence dans laquelle les quelconques parmi au moins 40 %, 50 % ou 60 % des nucléotides et/ou des analogues de nucléotides sont chimiquement modifiés.

8. Molécule de détection selon l'une quelconque des revendications 1 à 7, dans laquelle l'aptamère comprend au moins un nucléotide et/ou analogue de nucléotides avec un ou plusieurs résidus d'acides aminés attachés de manière covalente et/ou un ou plusieurs analogues d'acides aminés, de préférence dans laquelle un ou deux résidus d'acides aminés ou d'analogues d'acides aminés sont attachés de manière covalente sont attachés de manière covalente au nucléotide et/ou à l'analogue de nucléotides.

9. Molécule de détection selon l'une quelconque des revendications 1 à 8, dans laquelle le lieur comprend au moins 3 nucléotides, de préférence de 3 à 100 nucléotides et/ou analogues de nucléotides.

10. Molécule de détection selon l'une quelconque des revendications 1, 2, 4 à 9, dans laquelle le lieur permet l'association de l'aptamère et de la DNAzyme en l'absence d'une cible, de préférence dans laquelle le lieur comprend une séquence auto-complémentaire.

11. Molécule de détection selon l'une quelconque des revendications 1 à 10, dans laquelle la molécule de détection est une molécule d'ADN simple brin.

12. Molécule de détection selon l'une quelconque des revendications 1 à 11, dans laquelle la cible est sélectionnée dans le groupe constitué par les ions métalliques, les petites molécules organiques, les colorants organiques, les peptides, les protéines, les protéines de surface, les lipopolysaccharides, les cellules entières et les microorganismes.

13. Méthode de détection d'une molécule cible dans un échantillon, comprenant
(i) la mise en contact de la molécule de détection selon l'une quelconque des revendications 1 à 12 avec l'échantillon ;
(ii) la détermination de l'activité enzymatique de la DNAzyme, et
(iii) la détection de la molécule cible, dans laquelle l'activité enzymatique est une indication de la présence de la cible.

14. Méthode selon la revendication 13, dans laquelle l'échantillon est un échantillon industriel ou environnemental, de préférence un échantillon de terre, d'eau ou de flux de déchets ; ou un échantillon biologique, de préférence provenant de fluides corporels, d'écouvillons ou de tissus.

15. Utilisation in vitro d'une molécule de détection selon l'une quelconque des revendications 1 à 12 pour le diagnostic d'un trouble.
